# NEW EUROPEAN PATENT SPECIFICATION

(11) **EP 0 256 885 B2**
(45) Date of publication and mention of the opposition decision: **22.10.1997**
(45) Mention of the grant of the patent: 28.04.1993
(21) Application number: 87307308.4
(22) Date of filing: 18.08.1987
(51) Int. Cl.: A61L 15/00, A41B 1/02

(54) **Refastenable diapers and sheets therefor**
Wiederbefestigbare Windeln und Folien dafür
Couches pour bébés pouvant être refermées et feuilles pour cet usage

(30) Priority: 20.08.1986 US 898404
(43) Date of publication of application: 24.02.1988
(73) Proprietor: TREDEGAR INDUSTRIES, INC., Richmond, Virginia 23225 (US)
(72) Inventor: McBride, Robert Kimball, Jasonville Indiana 47438 (US)
(74) Representative: Baverstock, Michael George Douglas

(56) References cited:
- EP-A- 0 082 727
- EP-A- 0 143 924
- EP-A- 0 144 642
- EP-A- 0 253 792
- GB-A- 2 152 515
- GB-A- 2 152 516
- US-A- 3 137 672
- US-A- 3 355 520
- US-A- 4 366 292
- Procter & Gamble - Specifications - High strength polyethylene plastic film matte , finish March 6, 1984; page No 64467-469
- Hellerisch/Harsch/Haenle - Werkstoff-Führer Kunststoffe, 4. überarb. Auflage 1986, pages 144-146 and 156-160
- "Tabellarische Uebersicht über die Prüfung von Kunststoffen", B. Carlowitz , pages 14,15,20 and 21
- Franck/Biederbick, Kunststoff-Kompendium, Page 298

## Description

This invention relates to disposable diapers (and backsheets and compositions for use therein) having normally tacky and pressure-sensitive adhesive tape closures and is particularly concerned with composite closures (i.e. closures made with more than one tape) that can be opened and refastened without destroying either the diaper or the tape.

The invention especially relates to a thermoplastic monolayer film suitable for use as a refastenable diaper backsheet comprising a blend of polyolefins particularly polypropylene and polyethylene.

At least as early as 1955 it had been suggested to use strips of normally tacky and pressure-sensitive adhesive tape to hold conventional cloth diapers on an infant; see, e.g., US-A-2 714 889 and US-A-3 221 738. A few years later, when disposable diapers became extremely popular, strips of pressure-sensitive adhesive tape were again employed as closures; see, e.g., US-A-3 620 217.

A disposable diaper typically has a thin, flexible, stretchy low density polyethylene film cover, an absorbent filler on the inside of the cover, and a porous inner liner overlying the filler. Such a diaper is positioned at the crotch of an infant, the two ends of the diaper extending, respectively, toward the front and back. Adjacent edges of the diaper at each side are then either positioned next to each other or overlapped, a strip of pressure-sensitive adhesive tape being adhered to the cover at the border adjacent each of the two edges and holding the diaper closed. Because most pressure-sensitive adhesives bond firmly to the thin polyethylene diaper cover, it is almost impossible to open the tape closure without destroying the tape and/or the diaper cover in the process.

After a tape closure has been opened, it is frequently discovered that the diaper has not been soiled and hence that there is no need to replace it. If the cover has not been torn, a second strip of tape can sometimes be applied as a replacement closure, but this is often inconvenient. As a result, considerable work has been undertaken to develop a tape diaper closure that is not only capable of bonding firmly to the diaper cover, but is also capable of non-destructive removal and replacement. Closures of this type have generally involved a combination of two or more tapes, one of which remains permanently adhered to one edge of the diaper and is removably adhered to a so-called "target tape" mounted on the other edge of the diaper. Examples of such products are shown in US-A-3 951 149, US-A-3 987 793, US-A-3 999 546, and US-A-4 020 842.

The patents referred to in the preceding paragraph do not discuss the manner in which the closures are prepared. Typically in making such closures, the manufacturers of diapers mount rolls of the appropriate tape in their equipment, combining them to form a composite strip of tape, the width of which is substantially the same as the length of the diaper closure to be fabricated. The composite roll is then severed at right angles to the edges of the composite strip at intervals corresponding to the width of the desired tape closure and adhered at an appropriate location along the border adjacent the sides of the diaper. Although this manufacturing process is effective, many relatively small manufacturers are unable to provide the machinery necessary to accomplish the superimposition of several rolls of tape. As a result, it is important for a tape supplier to provide the manufacturers with a composite roll, made up of two or more specific tapes from which closures may readily be prepared.

A variety of diapers have been made with resealable tapes or refastenable disposable diapers. Representative are US-A-4 049 001, US-A-4 055 181, US-A-4 158 363, US-A-4 227 530, US-A-4 296 750, US-A-4 345 597, and US-A-4 369 786; EP-A-148 587; and DE-3 419 623.

US-A-4 330 888 discloses a disposable bib or napkin with an upper edge portion carrying a pressure-sensitive adhesive capable of releasably adhering to the clothing or body of a user. GB-A-1 320 628 and GB-A-1 342 115 disclose adhesive sheets or adhesive composite material which will releasably adhere to itself.

Additional diapers with tape fastening systems are described in US-A-3 646 937, US-A-3 848 594, US-A-3 874 386, US-A-3 951 149, US-A-3 987 793, US-A-3 999 546, US-A-4 014 339, US-A-4 014 340, US-A-4 020 842, US-A-4 041 949, US-A-4 047 004, US-A-4 194 507, US-A-4 207 895, US-A-4 209 016, US-A-4 237 889, US-A-4 573 986, and US-A-4 578 071.

EP-A-0 082 727 is concerned with embossed polyolefine films suitable for use as backsheets of a diaper of which the adhesiveness has been improved by arranging for the mean profile height of the surface to be less than 150 and the mean maximum peak valley height to be less than 230.

GB-A-2 152 515 describes blends of low density polyethylene and a major portion of linear low density polyethylene to which a small amount of polypropylene has been added with a view to improving stiffness but maintaining good optical properties, including good gloss, in films made therefrom.

EP-A-0 144 642 relates to multilayer film or sheet material having high adhesive bonding strength between the layers and good heat sealability comprising a first layer formed of a resin composition composed of a propylene-based resin and a linear-low density ethylene α-olefin copolymer having specified density and a second layer formed of a polypropylene-based resin or layer formed of a resin composition of a polypropylene-based resin and a low crystallinity α-olefin copolymer.

EP-A-0 253 792 was published after the priority date of the present Application and proposes film compositions having improved tape adhesiveness and suitable for use in baby diapers, comprising 0% to 95% of linear low density polyethylene, 0.1% to 50% of polypropylene and 0% to 95% by weight, low density polyethylene.

A wide variety of refastenable diapers have been constructed with varying degrees of success. One type of product has a one-piece pressure-sensitive tape tab system which permits multiple fastening and refastening of the pressure-sensitive diaper tape tab to the embossed thermoplastic film used as the outside or backsheet of the diaper.

The present invention provides a refastenable disposable diaper comprising a facing sheet, a backsheet, an absorbent panel positioned between said facing sheet and said backsheet and an adhesive tab fastener means attached to said back sheet, said backsheet being characterized by comprising a resin blend consisting essentially of polypropylene and a low density polyethylene which is LDPE, LLDPE, LMDPE or a mixture of any two of such low density polyethylenes in which from 10 to 20 weight percent of the resin blend is polypropylene, said backsheet having a transverse direction tensile strength value at 10 percent elongation of at least 515 grams, a gauge of 25.4 to 38.1 µm (1.0 to 1.5 mils), and a 120° tape adhesion value of at least 380 grams.

The present invention also provides use in refastenable disposable diapers of a backsheet consisting essentially of a blend of a low density polyethylene resin which is LDPE, LLDPE, LMDPE, or a mixture of any two of such low density polyethylene resins, a polypropylene resin, and a colour pigment, 10 to 20 weight percent of the resins in the blend being the polypropylene, said backsheet having a transverse direction tensile strength value at 10 percent elongation of at least 515 grams, a gauge of 25.4 to 38.1 µm (1.0 to 1.5 mils) and a 120° tape adhesion value of at least 380 grams.

An embossed monolayer thermoplastic film suitable for use as an outside sheet or backsheet of a refastenable disposable diaper in accord with the invention preferably has a transverse direction (T.D.) tensile strength (stress) value at low elongations (< 25%) between 0.454 Kg (one pound) and 1.362 Kg (three pounds); T.D. tensile strength @ break values above 0.908 Kg (two pounds); gloss @ 45 values (non-treated side) ≦ 8; slip C.O. F. (coefficient of friction) values (non-treated side) between 0.3 and 0.9 and tape peel force value between 0.454 Kg (one pound) and 0.908 Kg (two pounds) at nominal film thicknesses (≦ 38 µm (1.5 mils)).

A refastenable diaper sheet can be made using either blown-film or cast-film extrusion. Blown-film extrusion is preferred because films so made will be more balanced in the machine and transverse directions and have higher toughness values.

Non-refastenable embossed monolayer thermoplastic films used as the outside sheet or backsheet of a disposable diaper have been made largely of low density polyethylene (LDPE) resin blended with white pigments and slip additives as required to obtain the opacity (ash) and slip, coefficient of friction (C.O.F.) values needed. With a film thickness of about 38 µm (1.5 mils) such resin provides a suitable refastenable diaper backsheet. At film thicknesses of less than about 38 µm (1.5 mils), films of such resins have insufficient transverse direction (T.D.) tensile strength (stress) values at low elongations (≦25%) and consequently the film tends to tear or excessively distort as a pressure-sensitive diaper tab is peeled off. To boost the low T.D. tensile strength (stress) values, polyolefins such as high density polyethylene (HDPE) or medium density linear polyethylene (LMDPE) are added to the resin blend for making the film. Larger amounts of each (percent by weight) are required to obtain the desired stress value as nominal film thickness decreases.

Although these polyolefin blends may produce thermoplastic films of the desired T.D. tensile strength (stress) at low elongations, the film may have lowered tape adhesion, lowered drop dart impact (increased splittiness) and less than desired slip C.O.F.

At nominal film thicknesses of 25.4 - 38.1 µm (1.0-1.5 mils), conventional LDPE film extrusion equipment is detrimentally influenced by the high head pressure and torque requirements of the high loadings (percent by weight) of HDPE or LMDPE required to obtain the T.D. tensile strength (stress) values needed for refastenability.

It has been discovered that blending from ten weight percent to 20 weight percent polypropylene with LDPE, LLDPE, LMDPE, or mixtures of any two of the three resins, monolayer films can be produced which have the desired refastenability characteristic, i.e. increased T.D. tensile strength (stress) at elongations and increased tape adhesion without significant degradation of other film physical properties.

For monolayer films, the gauge of the film should be 25.4 to 38.1 µm (1.0 to 1.5 mils).

Some examples of monolayer thermoplastic films suitable for refastenable backsheets of disposable diapers are set forth in Table I.

**TABLE I**

| MONOLAYER FILMS | | | | | |
|---|---|---|---|---|---|
| RESIN BLEND (Percent by Weight) | | | | | Gauge (Mils) µm |
| P.P. | LDPE | LLDPE | LMDPE | White Concentrate | |
| 7 | 86 | | | 7 | (1.2) 30.5 |
| 5 | 88.5 | | | 6.5 | (1.2) 30.5 |
| 10 | 83 | | | 7 | (1.1) 27.9 |
| 10 | 83 | | | 7 | (1.2) 30.5 |
| 11 | 83 | | | 6 | (1.3) 33.0 |
| 12 | 83 | | | 5 | (1.4) 35.6 |
| 20 | 50 | 23 | | 7 | (1.1) 27.9 |
| 10 | 60 | | 25 | 5 | (1.4) 35.6 |
| 15 | 55 | | 25 | 5 | (1.4) 35.6 |
| 20 | 55 | | 20 | 5 | (1.4) 35.6 |
| 10 | 65 | | 20 | 5 | (1.4) 35.6 |
| 10 | 45 | | 40 | 5 | (1.4) 35.6 |
| 15 | 50 | | 30 | 5 | (1.4) 35.6 |
| P.P. = Polypropylene; 2 melt flow; .900 density; homopolymer LDPE = Low density polyethylene; 1.3 melt index; .925 density; liner grade LLDPE = Linear low density polyethylene; 1.0 melt index .917 density LMDPE = Linear medium density polyethylene; 1.0 melt index; .935 density | | | | | |

Coextruded or multilayer films are also suitable for use as a refastenable diaper backsheet. Such films should have a core layer of 50-100 percent polypropylene. The values for the monolayer films are applicable to the coating layers of coextruded films.

The core of a 25.4 µm (1.0 mil) coextruded film having 5.08 µm (0.2 mil) outer coatings is illustrated in Table II.

**TABLE II**

| COEXTRUDED OR MULTILAYER FILM | |
|---|---|
| RESIN BLEND (Percent by Weight) | |
| | Core 15.24 µm (0.6 Mil) |
| P.P. | 60 |
| LLDPE | 33 |
| White Concentrate | 7 |

In embossed monolayer films containing polypropylene in combination with linear low density polyethylene and/or linear medium density polyethylene, the advantages of each of the resins is obtained. High stress and high tape adhesion qualities of polypropylene are obtained and the high impact values and high tensile strength values of the linear polyethylenes are obtained, thereby providing a film with significantly better overall physical properties than those customarily used in diaper backsheets. Stress at low elongations (≦25 percent) and tape adhesion are also similarly improved in coextruded films containing polypropylene.

In Table III, a comparison is shown between LDPE film and films of various blends. It is readily seen that films containing polypropylene have increased adhesion values and increased T.D. stress @ 10%.

**TABLE III**

| T.D. STRESS AND 120° TAPE ADHESION VALUE FOR MIXED RESIN BLENDS | | | | | | |
|---|---|---|---|---|---|---|
| Blend (Percent by Weight) | | | | | T.D. Stress @ 10% (Grams) | 120° Tape Adhesion Value Grams |
| LDPE | LMDPE | HDPE | P.P. | Wh. Con. | | |
| 93 | | | | 7 | 400 | 295 |
| 83 | 10 | | | 7 | 464 | 286 |
| 88 | 5 | | | 7 | 470 | 329 |
| 83 | | 10 | | 7 | 525 | 351 |
| 73 | | 20 | | 7 | 650 | 348 |
| 88 | | | 5 | 7 | 515 | 380 |
| 83 | | | 10 | 7 | 560 | >425 |
| 73 | | | 20 | 7 | 804 | >425 |
| LDPE - Low Density Polyethylene LMDPE - Medium Density Linear Polyethylene HDPE - High Density Polyethylene P.P. - Polypropylene Wh. Con. - White Concentrate | | | | | | |

In Table IV, comparisons between similar films with and without polypropylene are illustrated. It is readily seen that films containing polypropylene have increased T.D. tensile strength (stress) at low elongations (≦25 percent) and increased tape adhesion.

**TABLE IV**

| T.D. TENSILE STRENGTH (STRESS) AND TAPE ADHESION OF COMPARABLE FILMS WITH AND WITHOUT POLYPROPYLENE | | | | | | |
|---|---|---|---|---|---|---|
| Blend (Weight Percent) | | | Embossing Pattern | Gauge (Mils) µm | T.D. Stress (Grams) | Tape Adhesion (Grams) |
| LDPE | P.P. | Wh. Con. | | | | |
| 93 | | 7 | FSII-B | (1.2) 30.5 | 651 | 417 |
| 86 | 7 | 7 | FSII-B | (1.2) 30.5 | 735 | 545 |
| 93 | | 7 | M-C | (1.2) 30.5 | 494 | 299 |
| 84 | 8 | 8* | M-C | (1.2) 30.5 | 617 | 425 |
| 93 | | 7 | FSII-B | (1.0) 25.4 | 586 | 294 |
| 83.5 | 9.5 | 7 | FSII-B | (1.0) 25.4 | 640 | 403 |
| 93 | | 7 | FSI-B | (1.35) 34.3 | 660 | 467 |
| 86.5 | 7 | 6.5 | FSI-B | (1.35) 34.3 | 800 | 571 |
| 93 | | 7 | M-C | (1.2) 30.5 | 606 | 296 |
| 86 | 7 | 7 | M-C | (1.2) 30.5 | 703 | 366 |

| | | | | | | |
|---|---|---|---|---|---|---|
| * Beige | | | | | | |
| FSII - Fine Square II FSI - Fine Square I B - Blown M - Matte C - Cast LDPE - Low Density Polyethylene P.P. - Polypropylene Wh. Con. - White Concentrate | | | | | | |

A disposable diaper comprises in general, a facing sheet defining a diaper inside surface for direction toward an infant, a moisture-impervious backsheet substantially coextensive with the facing sheet and defining a diaper outside surface, an absorbent panel positioned between the facing sheet and the backsheet, and an adhesive tab fastener means. On a refastenable or releasable tape closure, the tape or tab has a free end and a fixed end attached to the diaper. The free end of the tab has an adhesive which is adapted to be releasably affixed to the backsheet.

Refastenable diapers come in a variety of detailed constructions and releasable tapes. An example of such a diaper is illustrated in US-A-4 330 888.

The present invention provides a unique backsheet for such a refastenable disposable diaper.

A most preferred backsheet is constructed of a resin blend comprising by weight percent, about 12 percent polypropylene, about 83 percent low density polyethylene and about 5 percent white concentrate.

It can readily be appreciated that the instant invention can be incorporated in a variety of diaper constructions and releasable tape constructions. It is essential though that the diaper backsheet comprise or be made of sufficient polypropylene as to provide the desired strength and refastenability feature.

The foregoing disclosure and description of the invention is illustrative and explanatory thereof and various changes in the illustrated film or diaper construction may be made without departing from the scope of the invention as defined in appended claims.

## Claims (Claims for the following Contracting State(s): BE, DE, FR, GB, IT, LU, NL, SE)

1. A refastenable disposable diaper comprising a facing sheet, a backsheet, an absorbent panel positioned between said facing sheet and said backsheet and an adhesive tab fastener means attached to said backsheet, said backsheet being a coextruded multilayer film comprising (a) a polypropylene inner core of 50 to 100 percent polypropylene, and (b) coating layers each composed of a resin blend consisting essentially of polypropylene and a low density polyethylene which is LDPE, LLDPE, LMDPE or a mixture of any two of such low density polyethylenes in which from 10 to 20 weight percent of the resin blend is polypropylene, said backsheet having a transverse direction tensile strength value at 10 percent elongation of at least 515 grams, a gauge of 25.4 to 38.1 µm (1.0 to 1.5 mils), and a 120° tape adhesion value of at least 380 grams.

2. Use in refastenable disposable diapers of a backsheet composed of from 10 to 20 weight percent polypropylene, from 20 to 40 weight percent LMDPE, from 45 to 65 weight percent LDPE, and up to 5 percent by weight of opacifying pigment, said backsheet having a transverse direction tensile strength value to 10 percent elongation of at least 515 grams, a gauge of 25.4 to 38.1 µm (1.0 to 1.5 mils) and a 120° tape adhesion value of at least 380 grams.

## Claims (Claims for the following Contracting State(s): AT, CH, LI)

1. A refastenable disposable diaper comprising a facing sheet, a backsheet, an absorbent panel positioned between said facing sheet and said backsheet and an adhesive tab fastener means attached to said back sheet, said backsheet being characterized by comprising a resin blend consisting essentially of polypropylene and a low density polyethylene which is LDPE, LLDPE, LMDPE or a mixture of any two of such low density polyethylenes in which from 10 to 20 weight percent of the resin blend is polypropylene, said backsheet having a transverse direction tensile strength value at 10 percent elongation of at least 515 grams, a gauge of 25.4 to 38.1 µm (1.0 to 1.5 mils), and a 120° tape adhesion value of at least 380 grams.

2. A diaper as claimed in claim 1 in which the backsheet is a coextruded multilayer film comprising (a) a polypropylene inner core of 50 to 100 percent polypropylene, and (b) coating layers each composed of a resin blend of polypropylene and a low density polyethylene in which from 10 to 20 weight percent of the resin blend is polypropylene.

3. A diaper as claimed in claim 1 in which the backsheet is an embossed monolayer blown film made from a resin blend of low density polyethylene and polypropylene, the polypropylene constituting 10 to 20 weight percent of the blend.

4. Use in refastenable diapers of a backsheet consisting essentially of a blend of a low density polyethylene resin which is LDPE, LLDPE, LMDPE, or a mixture of any two of such low density polyethylene resins, a polypropylene resin, and a colour pigment, 10 to 20 weight percent of the resins in the blend being the polypropylene, said backsheet having a transverse direction tensile strength value at 10 percent elongation of at least 515 grams, a gauge of 25.4 to 38.1 µm (1.0 to 1.5 mils) and a 120° tape adhesion value of at least 380 grams.

5. Use as claimed in claim 4 wherein the backsheet is composed of from 10 to 20 weight percent polypropylene, from 20 to 40 weight percent LMDPE, from 45 to 65 weight percent LDPE, and up to 5 percent by weight of opacifying pigment.

## Patentansprüche (Patentansprüche für folgende(n) Vertragsstaat(en): BE, DE, FR, GB, IT, LU, NL, SE)

1. Wiederbefestigbare Wegwerfwindel, umfassend eine Anlageschicht, eine Unterlageschicht, eine zwischen der Anlageschicht und der Unterlageschicht angeordnete absorbierende Einlage und eine an der Unterlageschicht angebrachte Klebeband-Befestigungseinrichtung, welche Unterlageschicht eine koextrudierte mehrschichtige Folie ist, die (a) einen inneren Kern aus Polypropylen mit 50 bis 100 Prozent Polypropylen und (b) Überzugsschichten aufweist, von denen jede aus einer Harzmischung zusammengesetzt ist, die im wesentlichen aus Polypropylen und einem Polyethylen mit niedriger Dichte besteht, welches LDPE, LLDPE, LMDPE oder eine Mischung von zwei derartigen Polyethylenverbindungen mit niedriger Dichte ist, in welcher von 10 bis 20 Gewichtsprozent der Harzmischung Polypropylen ist, welche Unterlageschicht bei 10 Prozent Elongation in transversaler Richtung einen Zugfestigkeitswert von mindestens 515 g, eine Schichtstärke von 25,4 bis 38,1 µm (1,0 bis 1,5 mil) und einen 120°-Bandadhäsionswert von mindestens 380 g aufweist.

2. Verwendung einer Unterlageschicht in wiederbefestigbaren Wegwerfwindeln, welche Unterlageschicht aus 10 bis 20 Gewichtsprozent Polypropylen, von 20 bis 40 Gewichtsprozent LMDPE, von 45 bis 65 Gewichtsprozent LDPE und bis zu 5 Gewichtsprozent eines Opazität verleihenden Pigments zusammengesetzt ist, welche Unterlageschicht bei 10 Prozent Elongation in transversaler Richtung einen Zugfestigkeitswert von mindestens 515 g, eine Schichtstärke von 25,4 bis 38,1 µm (1,0 bis 1,5 mil) und einen 120°-Bandadhäsionswert von mindestens 380 g aufweist.

## Patentansprüche (Patentansprüche für folgende(n) Vertragsstaat(en): AT, CH, LI)

1. Wiederbefestigbare Wegwerfwindel, umfassend eine Anlageschicht, eine Unterlageschicht, eine zwischen der Anlageschicht und der Unterlageschicht angeordnete absorbierende Einlage und eine an der Unterlageschicht angebrachte Klebeband-Befestigungseinrichtung, welche Unterlageschicht dadurch gekennzeichnet ist, daß sie eine Harzmischung enthält, die im wesentlichen aus Polypropylen und einem Polyethylen mit niedriger Dichte besteht, welches LDPE, LLDPE, LMDPE oder eine Mischung von zwei derartigen Polyethylenen mit niedriger Dichte ist, in welcher von 10 bis 20 Gewichtsprozent der Harzmischung Polypropylen ist, welche Unterlageschicht bei 10 Prozent Elongation in transversaler Richtung einen Zugfestigkeitswert von mindestens 515 g, eine Schichtstärke von 25,4 bis 38,1 µm (1,0 bis 1,5 mil) und einen 120°-Bandadhäsionswert von mindestens 380 g aufweist.

2. Windel nach Anspruch 1, dadurch gekennzeichnet, daß die Unterlageschicht eine koextrudierte mehrschichtige Folie ist, die (a) einen inneren Kern aus Polypropylen mit 50 bis 100 Prozent Polypropylen und (b) Überzugsschichten aufweist, von denen jede aus einer Harzmischung aus Polypropylen und einem Polyethylen mit niedriger Dichte besteht, in welcher von 10 bis 20 Gewichtsprozent der Harzmischung Polypropylen ist.

3. Windel nach Anspruch 1, dadurch gekennzeichnet, daß die Unterlageschicht eine geprägte einschichtige Blasfolie ist, die aus einer Harzmischung aus Polypropylen mit niedriger Dichte und Polypropylen hergestellt ist, wobei das Polypropylen 10 bis 20 Gewichtsprozent der Mischung bildet.

4. Verwendung einer Unterlageschicht in wiederbefestigbaren Wegwerfwindeln, welche Unterlageschicht im wesentlichen aus einer Mischung aus einem Polyethylenharz mit niedriger Dichte, welches LDPE, LLDPE, LMDPE oder einer Mischung aus zwei derartigen Polyethylenharzen mit niedriger Dichte ist, einem Polypropylenharz und einem Farbpigment besteht, wobei 10 bis 20 Gewichtsprozent der Harze in der Mischung Polypropylen sind, welche Unterlageschicht bei 10 Prozent Elongation in transversaler Richtung einen Zugfestigkeitswert von mindestens 515 g, eine Schichtstärke von 25,4 bis 38,1 µm (1,0 bis 1,5 mil) und einen 120°-Bandadhäsionswert von mindestens 380 g aufweist.

5. Verwendung nach Anspruch 4, dadurch gekennzeichnet, daß die Unterlageschicht aus 10 bis 20 Gewichtsprozent Polypropylen, von 20 bis 40 Gewichtsprozent LMDPE, von 45 bis 65 Gewichtsprozent LDPE und bis zu 5 Gewichtsprozent eines Opazität verleihenden Pigments zusammengesetzt ist.

## Revendications (Revendications pour l'(les) Etat(s) contractant(s) suivant(s): BE, DE, FR, GB, IT, LU, NL, SE)

1. Couche jetable pour bébés, pouvant être rattachée, comprenant une feuille de contact, une feuille de support, une garniture absorbante positionnée entre ladite feuille de contact et ladite feuille de support, et un dispositif d'attache par pattes adhésives fixé à ladite feuille de support, ladite feuille de support étant un film multicouche coextrudé comprenant (a) une couche intérieure à base de polypropylène, comprenant 50 à 100 % de polypropylène, et (b) des couches de revêtement constituées chacune d'un mélange de résines consistant essentiellement en polypropylène et en un polyéthylène basse densité qui est le PEBD, le PELBD, le PELMD ou un mélange de deux quelconques de ces polyéthylènes basse densité, dans lequel 10 à 20 % en poids du mélange de résines sont constitués de polypropylène, ladite feuille de support ayant une valeur de résistance à la traction dans la direction transversale, à 10 % d'allongement, d'au moins 515 grammes, une épaisseur de 25,4 à 38,1 µm (1,0 à 1,5 mil) et une valeur d'adhérence de ruban adhésif à 120° d'au moins 380 grammes.

2. Utilisation dans des couches jetables pour bébés, pouvant être rattachées, d'une feuille de support constituée de 10 à 20 % en poids de polypropylène, de 20 à 40 % en poids de PELMD, de 45 à 65 % en poids de PEBD et d'une quantité allant jusqu'à 5 % en poids d'un pigment opacifiant, ladite feuille de support ayant une valeur de résistance à la traction dans la direction transversale, à 10 % d'allongement, d'au moins 515 grammes, une épaisseur de 25,4 à 38,1 µm, (1,0 à 1,5 mil), et une valeur d'adhérence de ruban adhésif à 120° d'au moins 380 grammes.

## Revendications (Revendications pour l'(les) Etat(s) contractant(s) suivant(s): AT, CH, LI)

1. Couche jetable pour bébés, pouvant être rattachée, comprenant une feuille de contact, une feuille de support, une garniture absorbante positionnée entre ladite feuille de contact et ladite feuille de support, et un dispositif d'attache par pattes adhésives fixé à ladite feuille de support, ladite feuille de support étant caractérisée en ce qu'elle comprend un mélange de résines consistant essentiellement en polypropylène et en un polyéthylène basse densité qui est le PEBD, le PELBD, le PELMD ou un mélange de deux quelconques de ces polyéthylènes basse densité, dans lequel 10 à 20 % en poids du mélange de résines sont constitués de polypropylène, ladite feuille de support ayant une valeur de résistance à la traction dans la direction transversale, à 10 % d'allongement, d'au moins 515 grammes, une épaisseur de 25,4 à 38,1 µm (1,0 à 1,5 mil), et une valeur d'adhérence de ruban adhésif à 120° d'au moins 380 grammes.

2. Couche pour bébés suivant la revendication 1, dans laquelle la feuille de support est un film multicouche coextrudé comprenant (a) une couche intérieure à base de polypropylène, comprenant 50 à 100 % de polypropylène, et (b) des couches de revêtement constituées chacune d'un mélange de résines consistant en polypropylène et en un polyéthylène basse densité, dans lequel 10 à 20 % en poids du mélange de résines consistent en polypropylène.

3. Couche pour bébés suivant la revendication 1, dans laquelle la feuille de support est un film soufflé monocouche gaufré constitué d'un mélange de résines consistant en un polyéthylène basse densité et en un polypropylène, le polypropylène représentant 10 à 20 % en poids du mélange.

4. Utilisation dans des couches pour bébés pouvant être rattachées, d'une feuille de support consistant essentiellement en un mélange d'une résine de polyéthylène basse densité qui est un PEBD, UN PELBD, un PELMD ou un mélange de deux quelconques de ces résines de polyéthylène basse densité, d'une résine de polypropylène et d'un pigment coloré, 10 à 20 % en poids des résines dans le mélange consistant en le polypropylène, ladite feuille de support ayant une valeur de résistance à la traction dans la direction transversale, à 10 % d'allongement, d'au moins 515 grammes, une épaisseur de 25,4 à 38,1 µm (1,0 à 1,5 mil) et une valeur d'adhérence de ruban adhésif à 120° d'au moins 380 grammes.

5. Utilisation suivant la revendication 4, dans laquelle la feuille de support est constituée de 10 à 20 % en poids d'un polypropylène, de 20 à 40 % en poids de PELMD, de 45 à 65 % en poids de PEBD et d'une quantité allant jusqu'à 5 % en poids d'un pigment opacifiant.
